(19) **Europäisches Patentamt**
**European Patent Office**
**Office européen des brevets**

(11) **EP 1 718 223 B1**

(12) **EUROPEAN PATENT SPECIFICATION**

(45) Date of publication and mention
of the grant of the patent:
**18.04.2007 Bulletin 2007/16**

(51) Int Cl.:
**A61B 17/17** (2006.01)

(21) Application number: **04713505.8**

(86) International application number:
**PCT/CH2004/000094**

(22) Date of filing: **23.02.2004**

(87) International publication number:
**WO 2005/079676 (01.09.2005 Gazette 2005/35)**

(54) **INTRAMEDULLARY NAIL**

MARKNAGEL

CLOU CENTROMEDULLAIRE

(84) Designated Contracting States:
**AT BE BG CH CY CZ DE DK EE ES FI FR GB GR HU IE IT LI LU MC NL PT RO SE SI SK TR**

(43) Date of publication of application:
**08.11.2006 Bulletin 2006/45**

(73) Proprietor: **Synthes GmbH**
**4436 Oberdorf (CH)**

(72) Inventors:
• **SCHLIENGER, André**
**CH-4144 Arlesheim (CH)**

• **BUETTLER, Markus**
**4702 Oensingen (CH)**
• **SENN, Peter**
**CH-4437 Waldenburg (CH)**

(74) Representative: **Lusuardi, Werther**
**Dr. Lusuardi AG,**
**Kreuzbühlstrasse 8**
**8008 Zürich (CH)**

(56) References cited:
**US-A- 5 041 115**     **US-A- 5 766 174**
**US-A- 6 010 506**     **US-A- 6 123 708**

**Description**

The invention relates to an intramedullary nail, in particular for the tibia, according to the preamble of claim 1.

Intramedullary nails with proximal and distal locking options are already known in the art. Depending on the fracture site and the anatomical situation, in particular for the tibia bone, an additional locking option on an intermediate level would be desirable. On this point, the invention intends to provide remedial measures. The invention is based on the objective of providing an intramedullary nail having an additional locking option, in the case of a tibial nail in the area of the Isthmus.

The invention solves the posed problem with an intramedullary nail that displays the features of claim 1.

The three level locking according to the invention optimizes the transmission of load by different distribution and transformation of the load applied to the nail. The additional intermediate locking level (between the proximal and distal level) serves as a support or artificial additional joint and therefore will divide the load on a new lever-arm-ratio. The intermediate level locking option may also serve in mechanical principle as an (elongated) Isthmus and will be the more efficient the bigger the gap between the nail diameter and the intramedullary canal of the bone is.

The various partial length of the intramedullary nail according to the invention are defined as follows:

$L_5$: proximal locking section extending from said proximal end to a distal boundary;

$L_6$: distal locking section extending from said distal end to a proximal boundary;

$L_7$: isthmus locking section located between said distal and proximal locking sections with a proximal boundary and a distal boundary;

$L_9$: distance between the proximal boundary of said isthmus locking section to said distal boundary of said proximal locking section.

$L_{10}$: distance of said distal boundary of said isthmus locking section to said proximal boundary of said distal locking section; and

$$L = (L_5 + L_9 + L_7 + L_{10} + L_6).$$

$L_D$ :distance between the distal end of the intramedullary nail to the through hole which is located nearest to said distal end.

$L_P$ : distance between the proximal end of the intramedullary nail to the through hole which is located nearest to said proximal end.

In a special embodiment the distinct intermediate sections of the intramedullary nail have no through-holes. This feature increases the stability in the fractured area.

In a further embodiment the intramedullary nail exhibits the following features:

A) a proximal locking section extending from said proximal end over the distance $0,22\,L < L_5 < 0,28\,L$ in direction of said distal end and having a distal boundary;

B) a distal locking section extending from said distal end over the distance $0,18\,L < L_6 < 0,22\,L$ in direction of said proximal end and having a proximal boundary; and

C) an isthmus locking section located between said distal and proximal locking sections with a proximal boundary and a distal boundary and a length of $0,08\,L < L_7 < 0,15\,L$.

The advantage obtained by these features is the creation of enough space to place sufficient locking options in different directions.

In a further embodiment the proximal boundary of said isthmus locking section has a distance $0,27\,L < L_9 < 0,33\,L$ to said distal boundary of said proximal locking section. This enables to locate the locking section at the level of the isthmus where there is the strongest bone and the most narrow part of the bone; this means with other words: less deviation of the nail axis to the bone axis at this position.

In a further embodiment said distal boundary of said isthmus locking section has a distance of $0,13\,L < L_{10} < 0,30\,L$ to said proximal boundary of said distal locking section. This feature allows to have a section free of locking holes where the surgeon can adjust the fracture line (in between the locking sections).

In a further embodiment the geometry of the intramedullary nail obeys to the condition: $0,32\,L < (L_{10} + L_6) < 0,50\,L$. That guarantees that the isthmus or intermediate locking section is in the area of the isthmus well away from the spongy bone.

In a further embodiment the intramedullary nail has a first intermediate section having the length $L_9$ between said proximal locking section and said isthmus locking section and which preferably has no through holes. This feature increases the strength of the intramedullary nail.

In a further embodiment the intramedullary nail has a second intermediate section between said distal locking section and said isthmus locking section having the length $L_{10}$ and which preferably has no through holes. This measure further increases the strength of the intramedullary nail.

In a further embodiment the isthmus locking section has two through holes, preferably arranged at a relative angle $\alpha$ in the range of $60° < \alpha < 120°$. This leads to a reduction of some degrees of freedom in the antero-posterior and medio-lateral direction.

In a further embodiment the through hole which is located nearest to said distal end of said intramedullary nail has a distance $L_D$ to said distal end in the range of $0{,}01 L < L_D < 0{,}38 L$.

In another embodiment the through hole which is located nearest to said proximal end has a distance $L_P$ to said proximal end in the range of $0{,}01 L < L_P < 0{,}70 L$.

In a further embodiment said proximal locking section having the length $L_5$ and said first intermediate section having the length $L_9$ are arranged at an angle $\beta$ in the range of $7° < \beta < 13°$. This configuration allows an easier insertion of the intramedullary nail into the bone and a proper alignment of the nail axis to the bone axis (biomechanical axis).

The invention and additional configurations of the invention are explained in even more detail with reference to the partially schematic illustration of several embodiments.

The invention is described below on the basis of advantageous embodiments and with reference to the attached drawings in which:

Fig. 1 shows, in a longitudinal view, an intramedullary nail according to the invention;

Fig. 2 shows an antero-posterior (or frontal) view of a modified intramedullary nail according to the invention inserted in a schematically represented tibia bone;

Fig. 3 shows the intramedullary nail of Fig. 2 in a latero-medial (or sagittal) view;

Fig. 4 shows an antero-posterior (or frontal) view of an intramedullary nail according to prior art with only two level locking;

Fig. 5 shows the intramedullary nail according to prior art of Fig. 4 in a latero-medial view; and

Fig. 6 shows an orthogonal section of the nail according to Fig. 1 along the line VI- VI in the region of one of the locking holes.

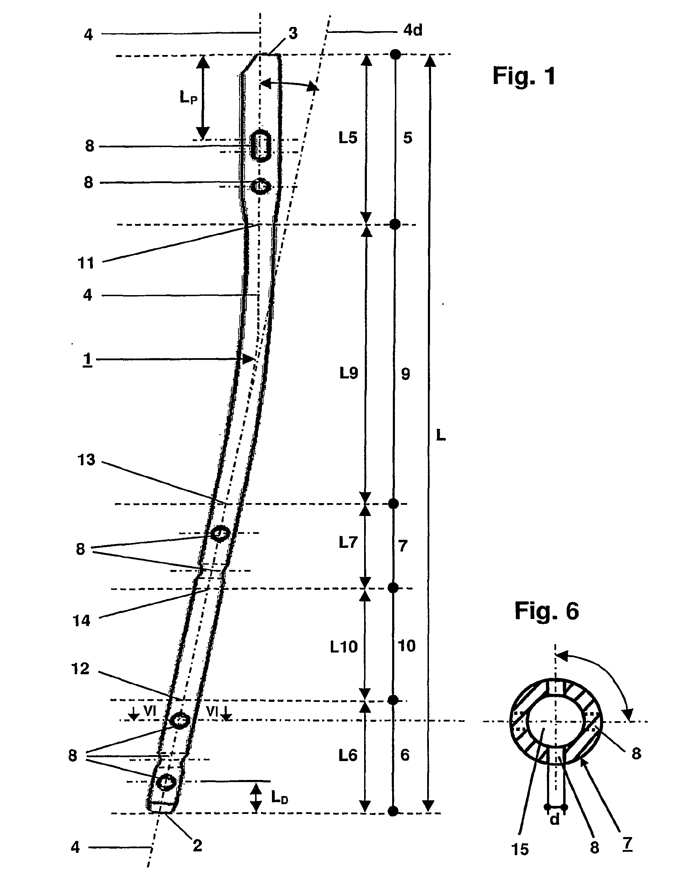

The intramedullary nail 1 shown in Fig. 1 has a distal end 2 for insertion into the medullary canal, a proximal end 3, a central axis 4 and a generally rod-like shape over the hole length L. The intramedullary nail 1 has three distinct locking sections 5,6,7. Each of these locking sections 5,6,7 has a number of through-holes 8 for receiving locking screws. Proximal locking section 5 has two through holes 8, one of which is an elongated one. Distal locking section 6 has three trough holes 8 two of which are parallel and the intermediate third through hole is running vertical to the other two. As shown in Fig. 6 the isthmus locking section 7 has two through holes 8 which are disposed at an angle $\alpha$ of 90°. Said three locking sections 5,6,7 are separated from each other by two distinct intermediate sections 9,10 having no through-holes 8.

The proximal locking section 5 extends from said proximal end 3 of said intramedullary nail 1 over a distance $L_5$ equal to $0{,}25 L$ in direction of said distal end 2 to a distal boundary 11. The distal locking section 6 extends from said distal end 2 over the distance $L_6$ equal to $0{,}2 L$ in direction of said proximal end 3 to a proximal boundary 12 and the isthmus locking section 7 located between said distal and proximal locking sections 5,6 with a proximal boundary 13 and a distal boundary 14 has a length of $L_7$ equal to $0{,}115 L$. Said proximal boundary 13 of said isthmus locking section 7 has a distance $L_9$ equal to $0{,}3 L$ to said distal boundary 11 of said proximal locking section 5. Said distal boundary 14 of said isthmus locking section 7 has a distance $L_{10}$ equal to $0{,}215 L$ to said proximal boundary 12 of said distal locking section 6. Therefore the sum of the two distances $L_{10} + L_6$ is equal to $0{,}415 L$.

The intramedullary nail 1 further has a first intermediate section 9 having the length $L_9$ between said proximal locking section 5 and said isthmus locking section 7 and which has no through holes 8. It further has a second intermediate section 10 between said distal locking section 6 and said isthmus locking section 7 having the length $L_{10}$ and which has no through holes 8.

The through hole 8 which is located nearest to said distal end 2 has a distance $L_D$ to said distal end 2 which is equal at least the diameter "d" of said through hole 8.

Said proximal locking section 5 having the length $L_5$ and said first intermediate section 9 having the length $L_9$ are arranged at an angle $\beta$ equal to 10° being the angle between the prolongation 4d of the central axis 4 in the distal locking section 6 and the central axis 4 in the proximal locking section 5.

As shown in Fig. 6 the intramedullary nail 1 has a cannulation 15 coaxial to the central axis 4.

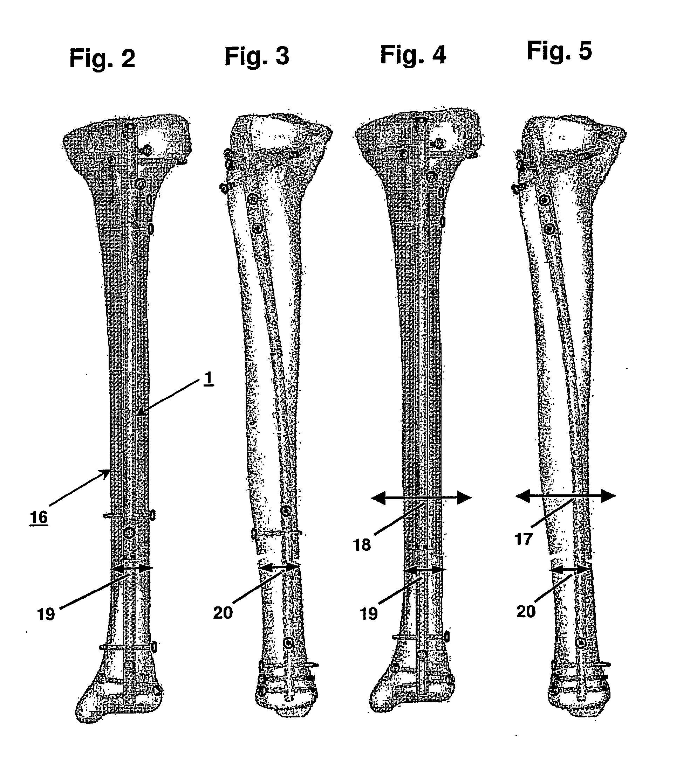

Figures 2 and 3 show the intramedullary nail 1 according to the invention implanted in a tibia bone with a distal fracture. In Fig. 2 the intramedullary nail 1 is shown in the antero-posterior view with several locking screws at three distinct levels. The fracture line is below the isthmus locking level. The same situation is represented in Fig. 3 in a sagittal view. Arrows 19 and 20 in Figs. 2 and 3 indicate the range of freedom for the distal bone fragment of the treated bone which is similar to the degree of freedom of a conventional intramedullary nail (Fig. 4 and 5) whereas the proximal fragment has a reduced freedom compared to a conventional nail due to its isthmus locking section. If the fracture line is above the isthmus section the distal fragment has a reduced degree of freedom.

Figures 4 and 5 show a conventional intramedullary nail with no isthmus locking section. Figure 4 represents an antero-posterior view and figure 5 a sagittal view. Arrows 17 and 18 in Figs. 4 and 5 indicate the range of freedom for the proximal bone fragment of the treated bone without an isthmus locking section which is much larger compared to the intramedullary nail 1 according to the invention represented in figures 2 and 3.

**Claims**

1. Intramedullary nail (1) with a distal end (2) for insertion into the medullary canal, a proximal end (3), a central axis (4) and a generally rod-like shape over the whole length L,
   **characterized in that**

   A) said nail (1) has three distinct locking sections (5,6,7) with at least one through-hole (8) each for receiving locking screws whereby said three locking sections (5,6,7) are separated from each other by two distinct intermediate sections (9,10) having less through-holes (8) per length unit than each of said locking sections (5,6,7); and
   B) said proximal locking section (5) having the length $L_5$ and said first intermediate section (9)-having the length $L_9$ are arranged at an angle $\beta$ in the range of $7° < \beta < 13°$.

2. Intramedullary nail (1) according to claim 1, **characterized in that** said distinct intermediate sections (9,10) have no through-holes (8)

3. Intramedullary nail (1) according to claim 1 or 2, **characterized by**

   A) a proximal locking section (5) extending from said proximal end (3) over the distance $0,22 L < L_5 < 0,28 L$ in direction of said distal end (2) and having a distal boundary (11);
   B) a distal locking section (6) extending from said distal end (2) over the distance $0,18 L < L_6 < 0,22 L$ in direction of said proximal end (3) and having a proximal boundary (12); and
   C) an isthmus locking section (7) located between said distal and proximal locking sections (5,6) with a proximal boundary (13) and a distal boundary (14) and a length of $0,08 L < L_7 < 0,15 L$.

4. Intramedullary nail (1) according to claim 3, **characterized in that** said proximal boundary (13) of said isthmus locking section (7) has a distance $0,27 L < L_9 < 0,33 L$ to said distal boundary (11) of said proximal locking section (5).

5. Intramedullary nail (1) according to claim 3 or 4, **characterized in that** said distal boundary (14) of said isthmus locking section (7) has a distance $0,13 L < L_{10} < 0,30 L$ to said proximal boundary (12) of said distal locking section (6).

6. intramedullary nail (1) according to one of the claims 3 to 5, **characterized in that** $0,32 L < (L_{10} + L_6) < 0,50 L$.

7. Intramedullary nail (1) according to one of the claims 1 to 6, **characterized in that** it has a first intermediate section (9) having the length $L_9$ between said proximal locking section (5) and said isthmus locking section (7) and preferably having no through holes (8).

8. Intramedullary nail (1) according to one of the claims 1 to 7, **characterized in that** said it has a second intermediate section (10) between said distal locking section (6) and said isthmus locking section (7) having the length $L_{10}$ and preferably having no through holes (8).

9. Intramedullary nail (1) according to one of the claims 1 to 8, **characterized in that** said isthmus locking section (7) has two through holes (8), preferably arranged at a relative angle $\alpha$ in the range of $60° < \alpha < 120°$.

**10.** Intramedullary nail (1) according to one of the claims 1 to 9, **characterized in that** the through hole (8) which is located nearest to said distal end (2) has a distance $L_D$ to said distal end (2) in the range of $0{,}01\,L < L_D < 0{,}38\,L$.

**11.** Intramedullary nail (1) according to one of the claims 1 to 10, **characterized in that** the through hole (8) which is located nearest to said proximal end (3) has a distance $L_P$ to said proximal end (3) in the range of $0{,}01\,L < L_P < 0{,}70\,L$.

**Patentansprüche**

**1.** Marknagel (1) mit einem distalen Ende (2) zum Einführen in den Markraum, einem proximalen Ende (3), einer Zentralachse (4) und einer im allgemeinen stabförmigen Form auf der gesamten Länge L, **dadurch gekennzeichnet, dass**

A) der genannte Marknagel (1) drei unterschiedliche Verriegelungsabschnitte (5,6,7) mit je mindestens einem Durchgangsloch (8) zur Aufnahme von Verriegelungsschrauben aufweist, wobei die genannten drei Verriegelungsabschnitte (5,6,7) durch zwei unterschiedliche, eine geringere Anzahl Durchgangslöcher (8) pro Längeneinheit als jeder der genannten Verriegelungsabschnitte (5,6,7) aufweisende Zwischenabschnitte (9,10) voneinander getrennt sind; und

B) der genannte, eine Länge $L_5$ aufweisende proximale Verriegelungsabschnitt (5) und der genannte, eine Länge $L_9$ aufweisende erste Zwischenabschnitt (9) in einem Winkel $\beta$ im Bereich von $7° < \beta < 13°$ angeordnet sind.

**2.** Marknagel (1) nach Anspruch 1, **dadurch gekennzeichnet, dass** die genannten unterschiedlichen Zwischenabschnitte (9,10) keine Durchgangslöcher (8) aufweisen.

**3.** Marknagel (1) nach Anspruch 1 oder 2, **gekennzeichnet durch**

A) einen proximalen Verriegelungsabschnitt (5), welcher sich vom genannten proximalen Ende (3) über eine Länge $0{,}22\,L < L_5 < 0{,}28\,L$ in Richtung des genannten distalen Endes (2) erstreckt und eine distale Abgrenzung (11) aufweist;
B) einen distalen Verriegelungsabschnitt (6), welcher sich vom genannten distalen Ende (2) über eine Länge $0{,}18\,L < L_6 < 0{,}22\,L$ in Richtung des genannten proximalen Endes (3) erstreckt und eine proximale Abgrenzung (12) aufweist; und
C) einen Isthmus-Verriegelungsabschnitt (7), welcher zwischen den genannten distalen und proximalen Verriegelungsabschnitten (5,6) angeordnet ist und eine proximale Abgrenzung (13), eine distale Abgrenzung (14) und eine Länge von $0{,}08\,L < L_7 < 0{,}15\,L$ aufweist.

**4.** Marknagel (1) nach Anspruch 3, **dadurch gekennzeichnet, dass** die genannte proximale Abgrenzung (13) des genannten Isthmus-Verriegelungsabschnittes (7) einen Abstand $0{,}27\,L < L_9 < 0{,}33\,L$ zur genannten distalen Abgrenzung (11) des genannten proximalen Verriegelungsabschnittes (5) aufweist.

**5.** Marknagel (1) nach Anspruch 3 oder 4, **dadurch gekennzeichnet, dass** die genannte distale Abgrenzung (14) des genannten Isthmus-Verriegelungsabschnittes (7) einen Abstand $0{,}13\,L < L_{10} < 0{,}30\,L$ zur genannten proximalen Abgrenzung (12) des genannten distalen Verriegelungsabschnittes (6) aufweist.

**6.** Marknagel (1) nach einem der Ansprüche 3 bis 5, **dadurch gekennzeichnet, dass** $0{,}32\,L < (L_{10} + L_6) < 0{,}50\,L$.

**7.** Marknagel (1) nach einem der Ansprüche 1 bis 6, **dadurch gekennzeichnet, dass** er zwischen dem genannten proximalen Verriegelungsabschnitt (5) und dem genannten Isthmus-Verriegelungsabschnitt (7) einen ersten Zwischenabschnitt (9) hat, welcher die Länge $L_9$ aufweist und vorzugsweise keine Durchganglöcher (8) hat.

**8.** Marknagel (1) nach einem der Ansprüche 1 bis 7, **dadurch gekennzeichnet, dass** er zwischen dem genannten distalen Verriegelungsabschnitt (6) und dem genannten Isthmus-Verriegelungsabschnitt (7) einen zweiten Zwischenabschnitt (10) hat, welcher die Länge $L_{10}$ aufweist und vorzugsweise keine Durchgangslöcher (8) hat.

**9.** Marknagel (1) nach einem der Ansprüche 1 bis 8, **dadurch gekennzeichnet, dass** der genannte Isthmus-Verriegelungsabschnitt (7) zwei Durchgangslöcher (8) hat, welche vorzugsweise unter einem relativen Winkel $\alpha$ im Bereich von $60° < \alpha < 120°$ angeordnet sind.

**10.** Marknagel (1) nach einem der Ansprüche 1 bis 9, **dadurch gekennzeichnet, dass** das Durchgangsloch (8), welches am nächsten bei dem genannten distalen Ende (2) angeordnet ist, einen Abstand $L_D$ im Bereich von $0,01\,L < L_D < 0,38\,L$ zu dem genannten distalen Ende (2) aufweist.

**11.** Marknagel (1) nach einem der Ansprüche 1 bis 10, **dadurch gekennzeichnet, dass** das Durchgangsloch (8), welches am nächsten bei dem genannten proximalen Ende (3) angeordnet ist, einen Abstand $L_P$ im Bereich von $0,01\,L < L_P < 0,70\,L$ zu dem genannten proximalen Ende (3) aufweist.

**Revendications**

**1.** Clou intramédullaire (1) présentant une extrémité distale (2) pour une insertion dans le canal médullaire, une extrémité proximale (3), un axe central (4) et une forme généralement analogue à une tige sur toute la longueur L,
**caractérisé en ce que**

A) ledit clou (1) comprend trois sections de verrouillage distinctes (5, 6, 7) présentant chacune au moins un trou traversant (8) pour recevoir des vis de blocage de sorte que lesdites trois sections de verrouillage (5, 6, 7) sont séparées les unes des autres par deux sections intermédiaires distinctes (9, 10) comportant moins de trous traversants (8) par unité de longueur que chacune desdites sections de verrouillage (5, 6, 7) ; et
B) ladite section de verrouillage proximale (5) de longueur $L_5$ et ladite première section intermédiaire (9) de longueur $L_9$ sont disposées à un angle $\beta$ dans la gamme de $7° < \beta < 13°$.

**2.** Clou intramédullaire (1) selon la revendication 1, **caractérisé en ce que** lesdites sections intermédiaires distinctes (9, 10) ne comportent aucun trou traversant (8).

**3.** Clou intramédullaire (1) selon la revendication 1 ou 2, **caractérisé par**

A) une section de verrouillage proximale (5) s'étendant à partir de ladite extrémité proximale (3) sur la distance $0,22\,L < L_5 < 0,28\,L$ en direction de ladite extrémité distale (2) et ayant une limite distale (11) ;
B) une section de verrouillage distale (6) s'étendant à partir de ladite extrémité distale (2) sur la distance $0,18\,L < L_6 < 0,22\,L$ en direction de ladite extrémité proximale (3) et ayant une limite proximale (12) ; et
C) une section de verrouillage en isthme (7) située entre lesdites sections de verrouillage distale et proximale (5, 6) ayant une limite proximale (13) et une limite distale (14) et une longueur de $0,08\,L < L_7 < 0,15\,L$.

**4.** Clou intramédullaire (1) selon la revendication 3, **caractérisé en ce que** ladite limite proximale (13) de ladite section de verrouillage en isthme (7) a une distance $0,27\,L < L_9 < 0,33\,L$ jusqu'à ladite limite distale (11) de ladite section de verrouillage proximale (5).

**5.** Clou intramédullaire (1) selon la revendication 3 ou 4, **caractérisé en ce que** ladite limite distale (14) de ladite section de verrouillage en isthme (7) a une distance $0,13\,L < L_{10} < 0,30\,L$ jusqu'à ladite limite proximale (12) de ladite section de verrouillage distale (6).

**6.** Clou intramédullaire (1) selon l'une des revendications 3 à 5, **caractérisé en ce que** $0,32\,L < (L_{10} + L_6) < 0,50\,L$.

**7.** Clou intramédullaire (1) selon l'une des revendications 1 à 6, **caractérisé en ce qu'**il comprend une première section intermédiaire (9) de longueur $L_9$ entre ladite section de verrouillage proximale (5) et ladite section de verrouillage en isthme (7) et ne comportant de préférence aucun trou traversant (8).

**8.** Clou intramédullaire (1) selon l'une des revendications 1 à 7, **caractérisé en ce qu'**il comprend une deuxième section intermédiaire (10) entre ladite section de verrouillage distale (6) et ladite section de verrouillage en isthme (7) de longueur $L_{10}$ et ne comportant de préférence aucun trou traversant (8).

**9.** Clou intramédullaire (1) selon l'une des revendications 1 à 8, **caractérisé en ce que** ladite section de verrouillage en isthme (7) comporte deux trous traversants (8), situés de préférence à un angle relatif $\alpha$ dans la gamme de $60° < \alpha < 120°$.

**10.** Clou intramédullaire (1) selon l'une des revendications 1 à 9, **caractérisé en ce que** le trou traversant (8) qui est le plus proche de ladite extrémité distale (2) a une distance $L_D$ jusqu'à ladite extrémité distale (2) dans la gamme de 0,01 L < $L_D$ < 0,38 L.

11. Clou intramédullaire (1) selon l'une des revendications 1 à 10, **caractérisé en ce que** le trou traversant (8) qui est le plus proche de ladite extrémité proximale (3) a une distance $L_P$ jusqu'à ladite extrémité proximale (3) dans la gamme de 0,01 L < $L_P$ < 0,70 L.

Fig. 1

Fig. 6

**Fig. 2**  **Fig. 3**  **Fig. 4**  **Fig. 5**